# FASCICULE DE BREVET EUROPEEN

(11) **EP 0 682 940 B1**
(45) Date de publication et mention de la délivrance du brevet: **10.01.2001**
(21) Numéro de dépôt: 95401130.0
(22) Date de dépôt: 16.05.1995
(51) Int. Cl.: A61K 7/48

(54) **Composition cosmetique ou dermatologique sous forme d'huile gélifiée contenant un mélange d'acide hydroxy-12 stéarique ou dialkylamide de l'acide N-lauroylglutamique et d'un copolymère styrène/alcadiène hydrogéné**
Kosmetisches oder dermatologisches Präparat in Form eines gelierten Öls enthaltend einen Gemisch 12-Hydroxystearinsäure oder N-Laurylglutaminsäure Dialkylamid und ein hydriertes Styren-Alkadien Copolymer
Cosmetic or dermatologic composition presented as a gelified oil containing a mixture of 12-hydroxystearic acid or N-lauroylglutamic acid dialkylamide and a hydrogenated styrene-alkadiene copolymer

(30) Priorité: 16.05.1994 FR 9405940
(43) Date de publication de la demande: 22.11.1995
(73) Titulaire: L'OREAL, 75008 Paris (FR)
(72) Inventeur: Afriat, Isabelle, F-75014 Paris (FR); Fodor, Pierre, F-92380 Garches (FR); Pouget, Françoise, F-94120 Fontenay-sous-Bois (FR)
(74) Mandataire: Stalla-Bourdillon, Bernard

(56) Documents cités:
- EP-A- 0 461 497
- WO-A-93/23008
- CHEMICAL ABSTRACTS, vol. 101, no. 12, 17 Septembre 1984, Columbus, Ohio, US; abstract no. 97494d, page 397 ; & JP-A-5 982 310 (SHISEIDO)

## Description

La présente invention a pour objet une composition cosmétique et/ou dermatologique sous forme d'huile gélifiée dont l'agent gélifiant est constitué par un mélange d'acide hydroxy-12 stéarique ou d'un dialkylamide de l'acide N-lauroylglutamique et d'un copolymère styrène/alcadiène hydrogéné.

L'utilisation d'huiles en cosmétique et/ou en dermatologie est particulièrement recherchée dans la mesure où elles présentent de très intéressantes propriétés notamment des propriétés émollientes, nettoyantes ou démaquillantes. Toutefois, leur utilisation seule présente un inconvénient majeur du fait de leur extrême fluidité.

En vue de remédier à cet inconvénient, il a donc été proposé de gélifier ou d'épaissir les huiles cosmétiques en y incorporant diverses substances telles que par exemple des cires ou encore des sels métalliques d'acides gras. Toutefois, l'utilisation de ces épaissisants ne permet par d'obtenir des gels transparents de bonne consistance et présentant des propriétés cosmétiques acceptables.

En effet, ces agents épaississants ont tendance à opacifier les gels, ce qui n'est pas particulièrement recherché par les utilisatrices qui préfèrent utiliser des gels transparents.

Dans la demande JP-01-163 111, il a été proposé des huiles cosmétiques gélifiées contenant en tant qu'agent gélifiant de l'acide hydroxy-12 stéarique. Si cet agent gélifiant permet d'obtenir des gels de bonne transparence, ceux-ci présentent néanmoins l'inconvénient d'avoir une texture cassante et d'être difficilement préhensiles lors de l'application.

Par ailleurs, dans la demande GB 1.485.694, il a été suggéré, en tant qu'agent gélifiant d'huiles et notamment d'huiles cosmétiques, l'emploi de N-acylamino acides ou de leurs dérivés tels que des N-acylamino acide amides. Si l'utilisation de ces N-acylamino acide amides permet également d'obtenir des gels transparents, ceux-ci souffrent néanmoins des mêmes inconvénients que ceux obtenus à partir de l'acide hydroxy-12 stéarique et sont par ailleurs difficiles d'application sur la peau en raison de leur toucher granuleux.

Il a en outre été proposé dans la demande WO 93/23008 des sticks antiperspirants sous forme de gel associant un N-acylamino acide amide, en tant qu'agent gélifiant, à de l'acide hydroxy-12 stéarique. Une telle combinaison est décrite dans cette demande comme permettant notamment d'améliorer la dureté des sticks.

On a maintenant constaté de façon surprenante et inattendue que lorsque des agents gélifiants tels que l'acide hydroxy-12 stéarique ou un dialkylamide d'un acide N-acyl glutamique étaient utilisés en association avec un copolymère styrène/alcadiène hydrogéné, il était possible non seulement d'améliorer la transparence des gels mais encore de les rendre plus aisément préhensiles et d'être plus agréables à l'application en raison de leur homogénéité.

La présente invention a donc pour objet à titre de produit industriel nouveau une composition cosmétique et/ou dermatologique sous forme d'huile gélifiée caractérisée par le fait qu'elle est essentiellement constituée d'au moins une huile cosmétique et, contient, en tant qu'agent gélifiant, un mélange d'acide hydroxy-12 stéarique ou d'un dialkylamide de l'acide N-lauroylglutamique et d'un copolymère styrène/alcadiène hydrogéné.

Comme mentionné précédemment, l'acide hydroxy-12 stéarique est un composé connu. On peut utiliser selon l'invention celui commercialisé par la Société Ajinomoto sous la dénomination de "GP100" ou celui commercialisé par la Société RMC Belix sous la dénomination d'acide hydroxy-12 stéarique.

Les dialkylamides de l'acide N-lauroylglutamique peuvent être représentés par la formule générale suivante : 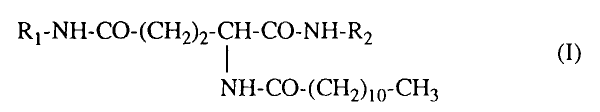 dans laquelle :
R₁ et R₂, identiques ou différents, représentent un radical alkyle, linéaire ou ramifié, saturé ou insaturé, ayant de 3 à 18 atomes de carbone.

Parmi les composés correspondants à la formule (I), on peut notamment citer le di-n-butylamide de l'acide N-lauroylglutamique, le di-(éthyl-2 hexylamide) de l'acide N-lauroylglutamique, le di-n-octylamide de l'acide N-lauroylglutamique et le di-stéarylamide de l'acide N-lauroylglutamique.

Selon un mode de réalisation préféré de l'invention, le dialkylamide de l'acide N-lauroylglutamique est le di-n-butylamide de l'acide N-lauroylglutamique vendu par la Société Ajinomoto Co., Inc., sous la dénomination de "GP1".

De façon générale, la concentration en acide hydroxy-12 stéarique ou en dialkylamide de l'acide N-lauroylglutamique de formule (I) dans l'huile gélifiée selon l'invention est de préférence comprise entre 0,1 et 2 % en poids, et notamment de 0,1 à 1,5 % en poids par rapport à son poids total.

Le copolymère styrène/alcadiène hydrogéné a de préférence un poids moléculaire supérieur à 20.000 et plus particulièrement inférieur à 150.000.

La proportion en poids du styrène par rapport à l'alcadiène, peut varier dans de larges limites comme par exemple entre 15/85 et 60/40. On préfère toutefois utiliser selon l'invention un rapport en poids d'environ 50/50. Selon l'invention le copolymére styrène/alcadiène hydrogéné est notamment le copolymère styrène/butadiène hydrogéné.

Comme copolymère styrène/butadiène hydrogéné, on peut utiliser selon l'invention celui vendu par la Société BASF sous la dénomination de "Luvitol HSB" ayant un poids moléculaire moyen d'environ 100.000.

La concentration en copolymère styrène/alcadiène hydrogéné dans l'huile gélifiée selon l'invention peut être comprise entre 0,1 et 20 % en poids et de préférence entre 3% et 12% en poids par rapport à son poids total.

Parmi les huiles cosmétiques pouvant être utilisées pour la réalisation des huiles gélifiées selon l'invention, on peut notamment citer :
- les hydrocarbures y compris les huiles minérales telles que les huiles de paraffine, les huiles de vaseline, les polyisobutènes hydrogénés tels que celui vendu par la Société Nippon Oil sous la dénomination de "Parleam" et les hydrocarbures ramifiés tels que ceux vendus par la Société Exxon Chemical sous la dénomination de "Isopar".
- les huiles végétales à base de triglycérides telles que l'huile de tournesol, l'huile de sésame, l'huile de colza, l'huile d'amande douce, l'huile de calophylum, l'huile de palme, l'huile d'avocat, l'huile de jojoba, l'huile d'olive, l'huile de ricin ou les huiles de germes de céréales telles que l'huile de germe de blé,
- les esters d'acides gras et d'alcools ou de polyols tels que les myristates d'isopropyle, de butyle ou de cétyle, les palmitates d'isopropyle, de butyle ou d'éthyl-2-hexyle, les stéarates d'isopropyle, de butyle, d'octyle, d'hexadécyle ou d'isocétyle, l'oléate de décyle, le laurate d'hexyle, l'huile de Purcellin vendue par la Société Dragoco qui est un mélange d'esters à chaîne ramifiée, les esters dérivés de l'acide lanolique tels que les lanolates d'isopropyle ou d'isocétyle, l'isononanoate d'isononyle, l'adipate de diisopropyle, le dicaprylate de propylène glycol, les octanoates et décanoates de glycol ou de glycérol ainsi que le ricinoléate de cétyle.

On peut également utiliser des mélanges d'esters benzoïques en C₁₂-C₁₅ vendus par la Société Witco sous la dénomination de "Finsolve TN".
- les huiles animales telles que le perhydrosqualène,
- les alcools à chaîne grasse tels que les alcools oléique, linoléique, linolénique et isostéarylique ou l'octyldodécanol.

On peut également utiliser en tant qu'huiles les mono-, di- ou triacétyl glycérines.

Selon un mode de réalisation particulier de l'invention, une partie des huiles hydrocarbonées telles que mentionnées ci-dessus peut être remplacée par des huiles de silicone telles que par exemple les polydiméthylpolysiloxanes, les phényldiméthicones, les cyclométhicones, les alkyldiméthicones, etc.

Selon ce mode de réalisation, la proportion en huile de silicone est telle que la transparence et la stabilité des gels ne soient pas modifiées. De préférence, la proportion en huile de silicone est comprise entre 1 et 10 % par rapport au poids total de l'huile gélifiée.

Selon un autre mode de réalisation de l'invention, on peut modifier les propriétés rhéologiques de l'huile gélifiée en ajoutant un correcteur de rhéologie dont le but principal est d'améliorer la préhensibilité du gel et sa facilité d'application sur la peau. Ce correcteur de rhéologie est un composé amphiphile comportant des groupements polaires susceptibles de former des liaisons hydrogènes ou des liaisons du type Van Der Waals ou susceptibles de former eux-mêmes des agrégats du fait de leur amphiphilie.

Parmi ces correcteurs de rhéologie des huiles gélifiées selon l'invention, on peut notamment citer :
- les alcools de Guerbet tels que le 2-butyloctanol,
- les alcools gras en C₁₂-C₁₈ oxyéthylénés tels que l'alcool cétylique oxyéthyléné à l'aide de 2 ou 20 moles d'oxyde d'éthylène, l'alcool laurique oxyéthyléné à l'aide de 25 moles d'oxyde d'éthylène, et l'alcool décylique oxyéthyléné à l'aide de 25 moles d'oxyde d'éthylène,
- les alcools de Guerbet polyoxyéthylénés,
- les silices hautement dispersées de granulométrie moyenne comprise entre 7 et 40 nm vendues par la Société Degussa sous la dénomination de "Aerosil".

L'agent correcteur de rhéologie est généralement utilisé à une concentration inférieure à 10 % en poids et de préférence comprise entre 0,01 et 3 % en poids par rapport au poids total de l'huile gélifiée.

Les huiles gélifiées selon l'invention peuvent également contenir divers ingrédients cosmétiques et en particulier des substances actives lipophiles telles que du tocophérol ou ses esters, des esters gras d'acide ascorbique, des céramides, des antioxydants, des anti-radicaux libres, des filtres absorbant ou réfléchissant le rayonnement UV, des parfums.

L'ensemble de ces ingrédients cosmétiques est généralement présent, dans l'huile gélifiée, à une concentration inférieure ou égale à 10 % en poids par rapport au poids total de l'huile gélifiée.

Les huiles gélifiées transparentes selon l'invention peuvent se présenter sous diverses formes en fonction notamment des ingrédients cosmétiques qu'elles contiennent. Il peut s'agir notamment de gels hydratants et émollients pour la peau à usage cosmétique et/ou dermatologique, de gels de démaquillage, de gels solaires ou de gels apaisants à usage cosmétique et/ou dermatologique ou encore de gels de coiffage pour les cheveux.

La préparation des huiles gélifiées selon l'invention est effectuée par solubilisation préalable du copolymère styrène/alcadiène hydrogéné dans l'huile ou le mélange d'huiles choisi sous vive agitation et à une température d'environ 80°C. On ajoute alors l'acide hydroxy-12 stéarique ou le dialkylamide de l'acide N-lauroylglutamique en élevant progressivement la température jusqu'à environ 100°C. Lorsque le mélange est devenu homogène, on ajoute éventuellement le correcteur de rhéologie puis laisse refroidir le mélange sous agitation modérée (environ 280 tr/mn) jusqu'à environ 25°C.

Lorsque l'huile gélifiée contient un ingrédient cosmétique liposoluble, celui-ci est ajouté, sous agitation, lors de l'étape de refroidissement.

L'invention a également pour objet l'utilisation d'une composition telle que définie précédemment dans le traitement cosmétique et/ou dermatologique de la peau, des ongles et/ou des cheveux.

L'invention a enfin pour objet un procédé de traitement cosmétique et/ou dermatologique consistant à appliquer sur la peau, les ongles et/ou les cheveux une composition cosmétique et/ou dermatologique telle que définie précédemment en vue d'en améliorer leur aspect esthétique.

On va maintenant donner à titre d'illustration plusieurs exemples de compositions cosmétiques et/ou dermatologiques sous forme d'huile gélifiée.

### EXEMPLE 1 : Huile démaquillante

On prépare selon l'invention une huile gélifiée démaquillante en procédant au mélange des ingrédients suivants :
- Acide hydroxy-12 stéarique 0,7 g
- Copolymère styrène/butadiène hydrogéné vendu sous la dénomination de "Luvitol HSB" 8,1 g
- Butyl-2 octanol 0,6 g
- Isononanoate d'isononyle 24,4 g
- Polyisobutène hydrogéné vendu sous la dénomination de "Parleam" q.s.p. 100 g

L'huile gélifiée obtenue est transparente et facilement préhensile. Le démaquillage se fait dans d'excellentes conditions.

### EXEMPLE 2 : Gel solaire

On prépare selon l'invention un gel solaire en procédant au mélange des ingrédients suivants :
- Acide hydroxy-12 stéarique 0,7 g
- Copolymère styrène/butadiène hydrogéné vendu sous la dénomination de "Luvitol HSB" 7,5 g
- Isononanoate d'isononyle 22,5 g
- Polydiméthylpolysiloxane vendu sous la dénomination de "Silicone L-45" par la Société Union Carbide 3 g
- p-Méthoxycinnamate d'éthyl-2 hexyle 3 g
- Vaseline 3 g
- Polyisobutène hydrogéné vendu sous la dénomination de "Parleam" q.s.p. 100 g

Ce gel huileux s'applique facilement sur la peau et permet de conférer une bonne protection vis-à-vis des radiations UV.

### EXEMPLE 3 : Gel émollient

On prépare selon l'invention un gel huileux émollient en procédant au mélange des ingrédients suivants :
- Di n-butylamide de l'acide N-lauroylglutamique vendu sous la dénomination de "GP1" par la Société Ajinomoto 0,9 g
- Copolymère styrène/butadiène hydrogéné vendu sous la dénomination de "Luvitol HSB" 7,38 g
- Butyl-2 octanol 0,6 g
- Isononanoate d'isononyle 22,12 g
- Silice vendue sous la dénomination de "Aérosil 200" par la Société Degussa 1,2 g
- Polyisobutène hydrogéné vendu sous la dénomination de "Parleam" q.s.p. 100 g

Ce gel huileux est transparent et facilement préhensile. Par application sur la peau, il confère d'excellentes propriétés émollientes.

## Revendications

1. Composition cosmétique et/ou dermatologique, sous forme d'huile gélifiée, caractérisée par le fait qu'elle est essentiellement constituée d'au moins une huile cosmétique et contient en tant qu'agent gélifiant, un mélange d'acide hydroxy-12 stéarique ou d'un dialkylamide de l'acide N-lauroylglutamique et d'un copolymère styrène/alcadiène hydrogéné.

2. Composition selon la revendication 1, caractérisée par le fait que le dialkylamide de l'acide N-lauroylglutamique correspond à la formule générale suivante : 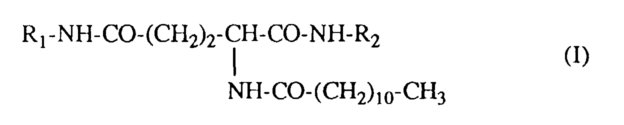 dans laquelle :
R₁ et R₂, identiques ou différents, représentent un radical alkyle, linéaire ou ramifié, saturé ou insaturé, ayant de 3 à 18 atomes de carbone.

3. Composition selon la revendication 1 ou 2, caractérisée par le fait que le dialkylamide de l'acide N-lauroylglutamique est choisi parmi le di-n-butylamide de l'acide N-lauroylglutamique, le di-(éthyl-2 hexylamide) de l'acide N-lauroylglutamique, le di-n-octylamide de l'acide N-lauroylglutamique et le di-stéarylamide de l'acide N-lauroylglutamique.

4. Composition selon l'une quelconque des revendications précédentes, caractérisée par le fait que la concentration en acide hydroxy-12 stéarique ou en dialkylamide de l'acide N-lauroylglutamique de formule (I) est comprise entre 0,1 et 2 % en poids par rapport au poids total de la composition.

5. Composition selon l'une quelconque des revendications précédentes, caractérisée par le fait que le copolymère styrène/alcadiène hydrogéné a un poids moléculaire supérieur à 20.000, la proportion en poids du styrène par rapport à l'alcadiène étant comprise entre 15/85 et 60/40.

6. Composition selon l'une quelconque des revendications précédentes, caractérisée par le fait que le copolymère styrène/alcadiène hydrogéné est un copolymère styrène/butadiène hydrogéné.

7. Composition selon la revendication 6, caractérisée par le fait que le copolymère styrène/butadiène hydrogéné a un poids moléculaire d'environ 100.000, la proportion en poids du styrène par rapport au butadiène étant d'environ 50/50.

8. Composition selon l'une quelconque des revendications précédentes, caractérisée par le fait que la concentration en copolymère styrène/alcadiène hydrogéné est comprise entre 0,1 à 20 % en poids par rapport au poids total de la composition.

9. Composition selon l'une quelconque des revendications précédentes, caractérisée par le fait qu'elle contient en outre un correcteur de rhéologie choisi parmi les alcools de Guerbet, les alcools gras en C₁₂-C₁₈ oxyéthélénés, les alcools de Guerbet polyoxyéthylénés et les silices hautement dispersées.

10. Composition selon la revendication 9, caractérisée par le fait que le correcteur de rhéologie est présent à une concentration inférieure à 10 % et de préférence comprise entre 0,01 et 3 % en poids par rapport au poids total de la composition.

11. Composition selon l'une quelconque des revendications précédentes, caractérisée par le fait qu'elle contient en outre des ingrédients cosmétiques lipophiles choisis parmi le tocophérol ou ses esters, les esters d'acide ascorbique, les céramides, les antioxydants, les anti-radicaux libres, les filtres absorbant ou réfléchissant le rayonnement UV et les parfums.

12. Composition selon la revendication 11, caractérisée par le fait que les ingrédients cosmétiques sont présents à une concentration inférieure ou égale à 10 % en poids par rapport au poids total de la composition.

13. Composition selon l'une quelconque des revendications précédentes, caractérisée par le fait qu'elle se présente sous forme d'un gel hydratant et émollient pour la peau, d'un gel de démaquillage, d'un gel solaire, d'un gel apaisant ou encore d'un gel de coiffage pour les cheveux.

14. Utilisation d'une composition selon l'une quelconque des revendications précédentes dans le traitement cosmétique de la peau, des ongles et/ou des cheveux.

15. Procédé de traitement cosmétique consistant à appliquer sur la peau, les ongles et/ou les cheveux une composition selon l'une quelconque des revendications 1 à 13 en vue d'en améliorer leur aspect esthétique.

## Patentansprüche

1. Kosmetische und/oder dermatologische Zubereitung in Form eines gelierten Öls, dadurch gekennzeichnet, dass sie im Wesentlichen aus mindestens einem kosmetischen Öl aufgebaut ist und als Geliermittel eine Mischung von 12-Hydroxystearinsäure oder eines Dialkylamids der N-Lauroylglutaminsäure und eines hydrierten Styrol/Alkadien-Copolymeren enthält.

2. Zubereitung gemäß Anspruch 1, dadurch gekennzeichnet, dass das Dialkylamid der N-Lauroylglutaminsäure der folgenden allgemeinen Formel entspricht: 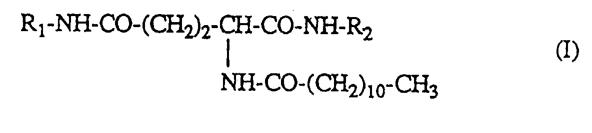 worin:
R₁ und R₂, die gleich oder verschieden sind, einen linearen oder verzweigten, gesättigten oder ungesättigten Alkylrest mit 3 bis 18 Kohlenstoffatomen darstellen.

3. Zubereitung gemäß Anspruch 1 oder 2, dadurch gekennzeichnet, dass das Dialkylamid der N-Lauroylglutaminsäure ausgewählt ist unter dem N-Lauroylglutaminsäure-di-n-butylamid, dem N-Lauroylglutaminsäure-di-(2-ethylhexylamid), dem N-Lauroylglutaminsäure-di-n-octylamid und dem N-Lauroylglutamirsäuredistearylamid.

4. Zubereitung gemäß einem der vorstehenden Ansprüche, dadurch gekennzeichnet, dass die Konzentration der 12-Hydroxystearinsäure oder des Dialkylamids der N-Lauroylglutaminsäure der Formel (I) zwischen 0,1 und 2 Gew.-% liegt, bezogen auf das Gesamtgewicht der Zubereitung.

5. Zubereitung gemäß einem der vorstehenden Ansprüche, dadurch gekennzeichnet, dass das hydrierte Styrol/Alkadien-Copolymer ein Molekulargewicht über 20 000 aufweist, wobei der Gewichtsanteil des Styrols bezogen auf das Alkadien zwischen 15/85 und 60/40 liegt.

6. Zubereitung gemäß einem der vorstehenden Ansprüche, dadurch gekennzeichnet, dass das hydrierte Styrol/Alkadien-Copolymer ein hydriertes Styrol/Butadien-Copolymer ist.

7. Zubereitung gemäß Anspruch 6, dadurch gekennzeichnet, dass das hydrierte Styrol/Butadien-Copolymer ein Molekulargewicht von etwa 100 000 aufweist, wobei der Gewichtsanteil des Styrols bezogen auf das Butadien etwa 50/50 ausmacht.

8. Zubereitung gemäß einem der vorstehenden Ansprüche, dadurch gekennzeichnet, dass die Konzentration des hydrierten Styrol/Alkadien-Copolymeren zwischen etwa 0,1 und 20 Gew.-% liegt, bezogen auf das Gesamtgewicht der Zubereitung.

9. Zubereitung gemäß einem der vorstehenden Ansprüche, dadurch gekennzeichnet, dass sie zusätzlich ein Korrekturmittel für die Rheologie enthält, ausgewählt unter den Guerbet-Alkoholen, ethoxylierten C₁₂-C₁₈-Fettalkoholen, polyethoxylierten Guerbet-Alkoholen und hochdispersen Silikaten.

10. Zubereitung gemäß Anspruch 9, dadurch gekennzeichnet, dass das Korrekturmittel für die Rheologie in einer Konzentration unter 10 % und vorzugsweise zwischen 0,01 und 3 Gew.-% vorhanden ist, bezogen auf das Gesamtgewicht der Zubereitung.

11. Zubereitung gemäß einem der vorstehenden Ansprüche, dadurch gekennzeichnet, dass sie zusätzlich lipophile kosmetische Bestandteile enthält, ausgewählt unter dem Tocopherol und dessen Estern, den Estern der Ascorbinsäure, den Ceramiden, den Antioxidanzien, den freien Radikalfängern (Mittel gegen freie Radikale), den die UV-Strahlung absorbierenden oder reflektierenden Filtern und den Parfümen.

12. Zubereitung gemäß Anspruch 11, dadurch gekennzeichnet, dass die kosmetischen Bestandteile in einer Konzentration von kleiner oder gleich 10 Gew.-% vorhanden sind, bezogen auf das Gesamtgewicht der Zubereitung.

13. Zubereitung gemäß einem der vorstehenden Ansprüche, dadurch gekennzeichnet, dass sie in Form eines hydratisierenden und weichmachenden Gels für die Haut, eines Abschminkgels, eines Sonnengels, eines beruhigenden Gels oder auch eines Gels zum Frisieren der Haare vorliegt.

14. Verwendung einer Zubereitung gemäß einem der vorstehenden Ansprüche bei der kosmetischen Behandlung der Haut, der Nägel und/oder der Haare.

15. Verfahren zur kosmetischen Behandlung, bestehend aus der Anwendung einer Zubereitung gemäß einem der Ansprüche 1 bis 13 auf der Haut, den Nägeln und/oder den Haaren zur Verbesserung des ästhetischen Erscheinungsbildes.

## Claims

1. Cosmetic and/or dermatological composition in the form of a gelled oil, characterized in that it consists essentially of at least one cosmetic oil and contains, as gelling agent, a mixture of 12-hydroxystearic acid or an N-lauroylglutamic acid dialkylamide and a hydrogenated styrene/alkadiene copolymer.

2. Composition according to Claim 1, characterized in that the N-lauroylglutamic acid dialkylamide corresponds to the following general formula: 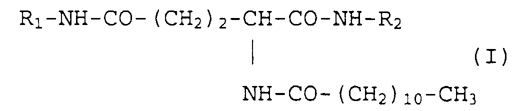 in which:
R₁ and R₂, which may be identical or different, represent a linear or branched, saturated or unsaturated alkyl radical containing from 3 to 18 carbon atoms.

3. Composition according to Claim 1 or 2, characterized in that the N-lauroylglutamic acid dialkylamide is chosen from N-lauroylglutamic acid di-n-butylamide, N-lauroylglutamic acid bis(2-ethylhexylamide), N-lauroylglutamic acid di-n-octylamide and N-lauroylglutamic acid distearylamide.

4. Composition according to any one of the preceding claims, characterized in that the concentration of 12-hydroxystearic acid or of N-lauroylglutamic acid dialkylamide of formula (I) is between 0.1% and 2% by weight relative to the total weight of the composition.

5. Composition according to any one of the preceding claims, characterized in that the hydrogenated styrene/alkadiene copolymer has a molecular weight of greater than 20,000, the weight proportion of styrene relative to the alkadiene being between 15/85 and 60/40.

6. Composition according to any one of the preceding claims, characterized in that the hydrogenated styrene/alkadiene copolymer is a hydrogenated styrene/butadiene copolymer.

7. Composition according to Claim 6, characterized in that the hydrogenated styrene/butadiene copolymer has a molecular weight of about 100,000, the weight proportion of styrene relative to butadiene being about 50/50.

8. Composition according to any one of the preceding claims, characterized in that the concentration of hydrogenated styrene/alkadiene copolymer is between 0.1% and 20% by weight relative to the total weight of the composition.

9. Composition according to any one of the preceding claims, characterized in that it also contains a rheology modifier chosen from Guerbet alcohols, oxyethylenated C₁₂-C₁₈ fatty alcohols, polyoxyethylenated Guerbet alcohols and highly dispersed silicas.

10. Composition according to Claim 9, characterized in that the rheology modifier is present in a concentration of less than 10% and preferably between 0.01% and 3% by weight relative to the total weight of the composition.

11. Composition according to any one of the preceding claims, characterized in that it also contains lipophilic cosmetic ingredients chosen from tocopherol or its esters, ascorbic acid esters, ceramides, antioxidants, free-radical scavengers, screening agents for absorbing or reflecting UV radiation, and fragrances.

12. Composition according to Claim 11, characterized in that the cosmetic ingredients are present at a concentration of less than or equal to 10% by weight relative to the total weight of the composition.

13. Composition according to any one of the preceding claims, characterized in that it is in the form of a moisturizing and emollient gel for the skin, a make-up-removing gel, an antisun gel, a soothing gel or a hair styling gel.

14. Use of a composition according to any one of the preceding claims in the cosmetic treatment of the skin, the nails and/or the hair.

15. Cosmetic treatment process which consists in applying a composition according to any one of Claims 1 to 13 to the skin, the nails and/or the hair in order to improve their aesthetic appearance.
